# EUROPÄISCHE PATENTSCHRIFT

(11) **EP 1 140 195 B1**
(45) Veröffentlichungstag und Bekanntmachung des Hinweises auf die Patenterteilung: **26.03.2003**
(21) Anmeldenummer: 99964607.8
(22) Anmeldetag: 18.12.1999
(51) Int. Cl.: A61K 47/10, A61P 39/06, A61K 9/107

(54) **EMULSION VOM TYP ÖL IN WASSER MIT SCHUTZWIRKUNG GEGEN PEROXIDATIONSSCHÄDEN AN MENSCHLICHEN ORGANEN, DEREN HERSTELLUNG UND VERWENDUNG**
OIL-IN-WATER EMULSION EXHIBITING A PROTECTIVE ACTION AGAINST DAMAGES TO HUMAN ORGANS RESULTING FROM PEROXIDATION, AND THE PRODUCTION AND USE THEREOF
EMULSION DE TYPE HUILE DANS EAU PRESENTANT UN EFFET PROTECTEUR VIS-A-VIS DES DEGRADATIONS PROVOQUEES PAR UNE PEROXYDATION SUR DES ORGANES HUMAINS, SA PREPARATION ET SON UTILISATION

(30) Priorität: 21.12.1998 DE 19859045
(43) Veröffentlichungstag der Anmeldung: 10.10.2001
(73) Patentinhaber: Fresenius Kabi Austria GmbH, 8055 Graz (AT)
(72) Erfinder: EGGENREICH, Udo, A-8020 Graz (AT); FEICHTINGER, Norbert, A-8010 Graz (AT); HOFER, Gerald, A-8141 Unterpremstätten (AT); SCHAUPP, Karin, A-8047 Graz (AT); SOMMERMEYER, Klaus, D-61191 Rosbach v.d.H. (DE); WURM, Anneliese, A-8010 Graz (AT); NAGEL, Eckhard, D-30900 Wedemark (DE); MEYER ZU VILSENDORF, Andreas, D-32257 Bünde (DE)
(74) Vertreter: Luderschmidt, Schüler & Partner GbR
(86) Internationale Anmeldenummer: EP9910106
(87) Internationale Veröffentlichungsnummer: WO00037108

(56) Entgegenhaltungen:
- WO-A-99/56776
- DE-A- 19 758 157
- US-A- 4 869 900

## Beschreibung

Die vorliegende Erfindung betrifft eine Öl in Wasser Emulsion, mit Schutzwirkung gegen Peroxidationsschäden an menschlichen Organen, mit einem Gehalt an α-Tocopherol, der Hauptkomponente von Vitamin E, als Wirkstoff, der jenen Gehalt an Vitamin E wesentlich übersteigt, wie er üblicherweise in Mulitvitaminlösungen enthalten ist. Diese Emulsion richtet sich besonders gegen eine Peroxidation die auftritt, wenn Organe einer Mangeldurchblutung oder gar einem völligen Durchblutungsstop ausgesetzt sind, auf die eine erneute Volldurchblutung folgt. Dieser Zustand kann bei jeder Art von Verschluß eines Blutgefäßes, das der Versorgung eines Organs dient, auftreten, wobei die erneute Durchblutung meist durch einen chirurgischen Eingriff wiederhergestellt wird. Sie wird aber im Zuge von Operationen an Organen, wie z.B. Niere, Leber, Darm oder Herz, auch bewußt herbeigeführt, indem das versorgende Blutgefäß abgeklemmt wird, um einen chirurgischen Eingriff an dem Organ vornehmen zu können. Nach beendeter Operation wird dann die Durchblutung wieder hergestellt. Ebenso kann diese Lösung mit gutem Erfolg im Zuge einer Organtransplantation eingesetzt werden. Die Applikation dieser Emulsion erfolgt in der Regel ausschließlich intravenös, insbesondere als Infusion. Sie ist aber auch als ergänzender Zusatz zu Organkonservierungslösungen im Zuge von Organtransplantationen geeignet.

Vitamin E ist ein essentielles, fettlösliches Vitamin, das schon in relativ niedrigen Konzentrationen Peroxylradikale von Lipiden wirksam bekämpfen kann. Es ist weiters hochwirksam im Schutz von Zellmembranen gegen Peroxidation, wobei auch hier unterstellt wird, daß diese Wirksamkeit ebenfalls auf dem Schutz vor einer Lipidoxidation beruht, also in der Zellmembran vorliegende Doppelbindungen von Lipiden gegen die Zerstörung durch Peroxidation geschützt werden. Dabei bleibt die Zellmembran in ihrer Struktur und Festigkeit intakt.

α-Tocopherol ist der Hauptbestandteil von Vitamin E. Unter den Begriff Vitamin E fallen auch die anderen Tocopherole wie β, y , und δ-Tocopherole, sowie weitere Tocol und Tocotrienolderivate. Das α-Tocopherol hat die Formel 2, 5, 7, 8 Tetramethyl-2- (4' 8' 12'trimethyltridecyl)chroman-6-ol, es hat in 2, sowie 4' und 8' Stellung 3 chirale Zentren und es gibt daher 8 stereoisomere Formen (RRR, SSS, RRS, SSR, RSR, SRS, RSS und SRR). In der Natur kommt jedoch nur die RRR-Form vor, die auch die stärkste Wirksamkeit besitzt. Daneben gibt es die anderen Stereoisomeren, die auf synthetischem Wege erhältlich sind, ebenso wie die Racematform, das all-rac-α-Tocopherol, das eine Mischung von gleichen Anteilen aller 8 Stereoisomerer ist. Es ist üblich, die Wirksamkeit aller stereoisomerer Formen, ebenso wie die der all-rac-Form auf die Wirksamkeit der RRR-Form zu beziehen. So entspricht beispielsweise die Wirksamkeit von 1 g des all-rac-α-Tocopherols der von 0,74 g des RRR-Stereoisomeren, jene von 1 g des SRR-Stereoisomeren der von 0,31 g RRR-α-Tocopherol. Die antioxidative Wirkung wird der phenolischen OH-Gruppe am Chromanring zugeschrieben.

Es ist üblich, Vitamin E im Rahmen von Multivitaminpräparaten peroral zu verabreichen, auch ist Vitamin E häufig in Form von Estern des a-Tocopherols in Multivitaminlösungen, die parenteral oder auch intravenös verabreichbar sind, enthalten, so daß damit ein etwaiger Vitamin-E-Mangel behoben bzw. einem solchen vorgebeugt werden kann.

In der WO 97/03651 wurden auch schon Öl in Wasser Emulsionen mit einem Gehalt an Vitamin E in der Ölphase beschrieben, wobei das Vitamin E als alleiniges Vitamin vorliegt, jedoch nicht den Wirkstoff der Öl in Wasser Emulsion darstellt. Wirkstoffe sind vielmehr schwerlösliche Stoffe, wie bevorzugt Itraconazol, ein Antimykoticum oder das Antikrebsmittel Taxol bzw. dessen Derivate, deren Löslichkeit in der Lipidphase durch den Zusatz von Vitamin E, insbesondere von α-Tocopherolen, verbessert werden soll. Ziel ist dabei, in der Lipidphase einen möglichst hohen Vitamin E Gehalt zu erzielen, wobei auch Emulsionen mit Vitamin E als alleinigem Bestandteil der Lipidphase inbegriffen sind. Diese Forderungen werden in erster Linie durch die Wahl von synthetischen Emulgatoren der Copolymerklasse (Poloxamere bzw. Pluronictypen) erfüllt. Hier können bei einem Gehalt von Lipiden in der Emulsion von 10 und 20 %, wobei diese Lipide aus einem Gemisch aus Sojaöl und Vitamin E im Gewichtsverhältnis von 1 : 1 bestehen, mit einem Gehalt von Poloxamer 407 bzw. Pluronic 127 in einer Menge von 2 bzw. 4 % stabile Emulsionen erhalten werden, die vor allem beim Emulgatorgehalt von 4 % hinsichtlich der Lösungsmöglichkeit für Taxol befriedigen. Diese synthetischen Emulgatoren konnten auch für die Herstellung von ölfreien Vitamin E Emulsionen eingesetzt werden. Werden hingegen Phospholipide, insbesondere Eilezithin, als Emulgator eingesetzt, werden Öl-Vitamin-E Verhältnisse von 1 : 1 in einer Emulsion, die ausreichend stabil ist, nicht erreicht und die Herstellung von Vitamin E als alleinigen Lipidstoff enthaltenden Emulsionen ist gemäß dieser Druckschrift nicht möglich, siehe dazu Beispiel 7, wonach bei Verwendung von Eilezithin als Emulgator bei einem Vitamin E-Gehalt von 10 oder 50 % und Eilezithingehalten von 0,4 bis 4 % keine stabilen Emulsionen erhalten werden konnten. Beispiele, bei denen mit Phospholipiden stabile Emulsionen erhalten wurden, weisen bei Verwendung des Sojaphosphatids Epikuron in allen Fällen einen Ölgehalt von 30 % und einen Vitamin E Gehalt von 5% aus, wobei der Gehalt an dem Sojaphosphatidgehalt 4 % betrug.

Das einzige Beispiel, bei dem Eilezithin als Emulgator diente, ist Beispiel 4, hier betrug, auf 1000 ml umgerechnet, der Gehalt an Sojaöl in der Emulsion 330 ml, jener an Vitamin E 80 g und jener an Eilezithin 66 g. Die i.v. Verabreichung solcher Emulsionen an empfindliche Patienten ist nicht denkbar.

Ein besonderes Problem stellen die Peroxidationsschäden dar, die im Falle von Ischämie und nachfolgender Reperfusion auftreten, weil es sich hier meist um schwerst kranke Patienten handelt, z.B.: solche, bei denen schwere Operationen an Leber, Herz oder Niere oder gar Organtransplantationen vorgenommen werden. Hier bestand die Meinung, daß diese Schäden durch die Gabe von Vitamin E gemildert werden könnten, falls diese auch auf eine Lipidperoxidation zurückzuführen sind. Man hat daher Tierversuche, z.B.: an Mäusen oder Ratten vorgenommen, um zu sehen, ob diese Schäden tatsächlich durch Vitamin E bekämpft werden können. In vielen Fällen, jedoch nicht immer, konnte an diesen Versuchen gezeigt werden, daß Peroxidationsschäden als Folge von Ischämie und/oder Reperfusion durch Gabe von Vitamin E bzw. α-Tocopherol vermindert werden können.

Unter mehreren Applikationsmöglichkeiten wie z.B. die orale oder intraperetoneale Gabe an die Versuchstiere, wurde auch die intravenöse Applikation an Versuchstieren untersucht. So wurde z.B. von Ikezawa T., Nishikimi N., Oba Y. in ihrer Arbeit "Lipidperoxides in the mechanism of ischemia/reperfusion injury in skeletal muscle; experimental studies" VASC SURG 1993, 27, 191 - 201 festgestellt, daß an einem Ischämie-Reperfusionsmodell an Skelettmuskeln von Hunden bei i.v. Gabe von 500 mg Vitamin E (all-rac-Tocopherolacetat, Präparat mit Polyethylenglykol 400), die über wenige Minuten unmittelbar vor der Reperfusion verabreicht wurden, der zu erwartende Ischämie-Reperfusionsschaden gelindert wurde. Dies äußerte sich u. a. darin, daß der signifikante Anstieg von Serum-Kreatin-Phosphokinase, der als ein Zeichen auftretender Rhabdomyolyse gilt, während der Reperfusionsphase durch die α-Tocopherolgabe unterdrückt wurde, sowie, daß durch diese Gabe der Anstieg der Lipidperoxide im Serum, gemessen als farbiges Reaktionsprodukt (TBARS, Thiobarbitursäure-Reaktive-Substanzen) der Reaktion von entstandenem Malondialdehyd (MDA) mit Thiobarbitursäure, verhindert wurde. Ferner konnte an Ratten, die Vitamin E sowohl oral über eine Schlundsonde, als auch intravenös über einen Zentralvenenkatheter über je 3 Tage in Dosen von 1,0 mg/Tag oral bzw. 0,5 mg/Tag intravenös verabreicht erhielten und dann einem oxidativen Streß ausgesetzt wurden, gezeigt werden, daß durch intravenöse Zufuhr eine signifikante Anreicherung von Vitamin E im Plasma und Aortenendothel gegenüber Kontrolltieren, die kein Vitamin E erhielten, erreicht werden konnte, wobei die erreichte Vitamin-E Konzentration im Plasma auch gegenüber jenen Tieren, die das Vitamin E oral in der doppelten Dosis erhielten, signifikant erhöht war. Diese Gabe an Vitamin E zeigte deutliche Effekte hinsichtlich des Grades der Lipidperoxidation. Die Konzentration an TBARS im Plasma war bei den Tieren, die Vitamin E intravenös erhielten, signifikant niedriger als im Plasma der Kontrolltiere und zeigte auch unter oxidativem Streß keine weitere Zunahme (Engelhart K., Fuerst P., Biesalski HK, Untersuchungen zur parenteralen Gabe von Vitamin E bei Ratten (Abstract) 16 DGEM-AKE Jahrestagung, Stuttgart-Hohenheim, 1997. Aktuelle Ernährungsmedizin 1997, 22 (1), 49, 50.

Nähere Details über die zu solchen Versuchen verwendete α-Tocopherolzubereitung finden sich in der Arbeit der gleichen Autoren K. Engelhart et al. Free Rad. Res., Vol. 29, pp. 421 - 426, 1998, wonach für die parenterale Verabreichung der α-Tocopherolzubereitung an die als Versuchstiere eingesetzten Ratten eine käufliche Fettemulsion verwendet wurde, der soviel all-rac-α-Tocopherol zugesetzt wurde, daß eine Konzentration an Tocopherol in der Emulsion von 12,5 mmol/l (=5,4 g/l) erzielt wurde. Die käufliche Fettemulsion enthielt pro 100 ml 10g Sojaöl, 1,2g Eilezithin, 2,5g Glycerin, 30 mg Ölsäure, 3mg NaOH und 4 µmol/l Vitamin E. Sie weist also pro Liter einen Gehalt an 100 g Sojaöl, 12 g Eilezithin, 25 g Glycerin, 300 mg Ölsäure und 30 mg Natronlauge auf. Vor der intravenösen Verabreichung an die als Versuchstiere verwendeten Ratten wurde diese Emulsion mit physiologischer Kochsalzlösung so weit verdünnt, dass eine Konzentration an all-rac-α-Tocopherol von 0,58 mmol/l, das sind 0,025 g/l, resultierte. Sie wurde den Ratten, wie schon in der oben angeführten Publikation von K. Engelhart, P. Fürstund H. K. Biesalski geschildert wird, über einen Zentralvenenkatheter über 3 Tage in Dosen von je 0,5 mg all-rac-α-Tocopherol/Tag intravenös verabreicht Als Resultat wurde in der Arbeit von Engelhart et al. in Free Rad. Research, Vol. 29 hinsichtlich Wirkung der Tocopherolapplikation nur eine signifikante Erhöhung des α-Tocopherolspiegels im zellassoziierten Aortenendothel beschrieben.

Schließlich wurde auch der Zusatz von Vitamin E zu Organkonservierungslösungen im Tierversuch untersucht. (A. Demirbas et al., Transplantation Proceedings 25 (3), (1993) 2274, und Silka Selliak et al., Ceylon Medical Journal 1995, 40, 97 - 100). In beiden Arbeiten zeigte die mit Vitamin E behandelte Niere nach erfolgter Transplantation geringere Peroxidation und bessere Nierenfunktion.

Als Vitamin E wurde bei A. Demirbas et al. α-Tocopherol eingesetzt, wie es verabreicht wurde, geht aus der Arbeit nicht hervor. Silka Selliak hingegen spezifizierte seine Vorgangsweise. Er fügte der Konservierungslösung "water soluble Vitamin E (Merck)" hinzu, das kein Tocopherol ist, sondern ein Bruchstück mit Vitamin E Wirkung (Trolox), bei dem der hydrophobe Teil fehlt.

Den Einfluß von antioxidativ wirkenden Vitaminen auf Peroxidationsschäden im Rahmen einer Organtransplantation untersuchten "Rabl H. Khoschsorur G., Colombo T., Petritsch P., Rauchenwald M., Koeltringer P., Tatzber F., Esterbauer H., "A multivitamin infusion prevents lipid peroxidation and improves transplantation performance". Kidney International 1993, 43 (4): 912 - 917." an Patienten, an denen eine Nierentransplantation durchgeführt wurde. Dabei lag die damals nach als Hypothese bezeichnete Meinung zugrunde, dass Schäden durch Ischämie und Reperfusion bei Nierentransplantationen mit einer Lipidperoxidation zusammenhängen könnten, und daher die Hemmung der Lipidperoxidation durch Antioxidantien zu einer Verbesserung der Funktion der transplantierten Niere führen müßte. Die Untersuchungen wurden an 30 Patienten durchgeführt. 16 dieser Patienten erhielten nach erfolgter Transplantation einer Niere, jedoch noch in der ischämischen Phase 30 Minuten vor der Reperfusion der Niere eine auf 500 ml verdünnte Lösung von 20 ml einer Multivitaminlösung, die unter dem Namen Omnibionta zugelassen war, infundiert. Mit dieser Lösung erhielt jeder Patient 10 mg α-Tocopherolacetat und 0,2 mg α-Tocopherol. Ferner enthielt die verabreichte Lösung noch Vitamin C, Vitamin A und 5 B-Vitamine, denen allen auch eine antioxidative Wirkung zugeschrieben wurde. Das verabreichte Multivitaminpräparat war eine Zubereitung auf wäßriger Basis. Sie enthielt als Cosolvens 300 mg Benzylalkohol, 1000 mg Polysorbat 80, 400 mg Propylenglykol, 5000 mg Glycerin 85 %, 720 mg Trometamol und Wasser bis zum Volumen von 20 ml. Diese Lösung wurde mit physiologischer Kochsalzlösung auf 500 ml verdünnt. Die Dosis an α-Tocopherol entsprach dabei nur dem empfohlenen täglichen Bedarf an Vitamin E, während Vitamin C in der 13-fachen Menge des täglichen Bedarfes und Vitamin A in der 6-fachen Menge des täglichen Bedarfes verabreicht wurden. Wie die Auswertung von Blutproben der Patienten anhand von Malondialdehydbestimmungen (MDA) im Plasma zum Zeitpunkt der Gabe der Vitaminlösung 30 Minuten vor der Reperfusion sowie 1, 2, 3 und 4 Stunden nach der Reperfusion ergab, konnte mit der Vitaminlösung eine Erhöhung der MDA-Werte verhindert werden, während bei den Kontrollpatienten eine deutliche Erhöhung der Werte, vor allem 1 Stunde nach Beginn der Reperfusion registriert wurde. Damit war der Beweis erbracht, daß die Verabreichung dieser Vitaminlösung die Lipidperoxidation während der Reperfusion gehemmt hatte. Gleichzeitig wurde anhand der Creatinin-Clearence eine Verbesserung der Nierenfunktion der transplantierten Niere bei jenen Patienten dokumentiert, die die Multivitaminlösung infundiert erhalten hatten. Die Verfasser schrieben dabei die wesentliche Wirkung dem Vitamin C zu, dessen Wirkung durch eine Schutzwirkung für Vitamin E das primäre Antioxidans, gegen Zerstörung durch freie Radikale, erklärt wurde und somit vermutlich ein Recycling von Vitamin E stattfand. Rabl et al. erwähnten auch, daß Omnibionta deshalb gewählt wurde, weil es die einzige in Österreich zugelassene Präparation mit antioxidativ wirksamen Vitaminen war. Es stellte also kein Mittel der Wahl dar. Dies liegt auch nahe, wenn man bedenkt, daß es sich um eine wäßrige Vitamin-Infusionslösung handelte, die zur Lösungsvermittlung der hydrophoben Bestandteile Substanzen wie Benzylalkohol, Propylenglykol und andere Zusätze benötigte, die die Verträglichkeit herabsetzen und zu Nebenwirkungen Anlaß geben. Solche Lösungen sind zur Verabreichung an Patienten während einer schweren Operation, also im Schockzustand, kaum geeignet. Schließlich ist die Tocopheroldosis sehr niedrig, wobei die Hauptmenge desselben als Acetat vorliegt, von dem bekannt ist, daß es nicht vollständig verwertet wird. Ferner ist festzustellen, daß mit der Infusion des Präparats erst nach erfolgter Transplantation, 30 Minuten vor der Reperfusion, begonnen wurde, wobei der Hauptteil der Infusion zu Beginn der Reperfusion zugeführt war. Der Erfolg der Verminderung von Peroxidationsschäden wurde dadurch ermittelt, daß der MDA-Wert 30 Minuten vor der Reperfusion, jedoch nach erfolgter Transplantation als Basiswert genommen wurde. Peroxidationsschäden sind jedoch nicht nur während der Reperfusion zu erwarten, sondern treten bereits während der Ischämie auf, zumal sich in der Ischämiephase Xanthinoxidase, Hypoxanthin und Xanthin in den Zellen unphysiologisch anreichern, wie in Tierversuchen festgestellt wurde. Rabl et al. haben ausdrücklich darauf hingewiesen. Die Peroxidationsschäden während der Reperfusion sind großteils auf die Anreicherung dieser Verbindungen, die mit dem zugeführten molekularen Sauerstoff unter Bildung von Peroxyverbindungen und Radikalen reagieren, zurückzuführen. Ein Schutz auch während der Ischämie-Phase wäre daher angebracht.

Es bestand daher die Aufgabe, eine für die i.v. Applikation in Form einer Infusion geeignete Darreichungsform von α-Tocopherol zu finden, die insbesondere auch für Patienten unter Schock ausreichend verträglich ist. Sie soll die Verabreichung einer vorzugsweise einmaligen Tocopheroldosis in einer Höhe ermöglichen, die imstande ist, sowohl die Reperfusion als auch die Phase der Ischämie abzudecken. Das bedeutet, daß sie sich durch ein hohes Maß an Verträglichkeit auszeichnen muß.

Dies gelingt erfindungsgemäß dadurch, daß a-Tocopherol nur als solches, nicht aber in Esterform verabreicht wird, die Infusion als eine Öl in Wasser Emulsion formuliert ist, die jedoch einen Fettgehalt von maximal 2,5 % Gewicht/Volumen aufweist, bei der Phospholipide, insbesondere Eilezithin, als Emulgator dienen, die ebenfalls in beschränkter Menge, nämlich in einer solchen von max. 0,3 % eingesetzt werden, und die RRR-α-Tocopherol, all-rac-α-Tocopherol oder andere Stereoisomere α-Tocopherole in einer Konzentration enthält, die hinsichtlich Wirkung jener von mindestens 1,48 g/l RRR-α-Tocopherol entspricht.

In besonders kritisch gelagerten Fällen kann auf den Fettgehalt überhaupt verzichtet werden, d.h., daß das Tocopherol die einzige Ölkomponente darstellt. Fettemulsionen mit Phospholipiden, insbesondere mit Eilezithin als Emulgator, sind wegen ihrer guten Verträglichkeit bekannt und synthetischen Emulgatoren überlegen, da sie den Chylomicronen ähnlich sind. Diese an sich schon gute Verträglichkeit wird durch die erfindungsgemäßen Maßnahmen noch erhöht. Sie sind daher auch Systemen auf Basis Wasser und organischen Lösungsmitteln hinsichtlich i.v. Verträglichkeit deutlich überlegen.

Bei Kenntnis der Lehre gemäß WO 97/03651 Danbiosyst UK LTD, war es nicht zu erwarten, daß sich stabile Emulsionen mit α-Tocopherol als Wirkstoff und Phospholipiden als Emulgator mit den erfindungsgemäß niedrigen Öl- und Emulgatormengen herstellen lassen, wobei es besonders überraschend ist, daß eine Erhöhung der Menge an α-Tocopherol pro Volumseinheit auf das 5-fache keiner Erhöhung der Öl- und Emulgatormenge bedurfte. Bezüglich der Herstellung von α-Tocopherol Emulsionen ohne Zusatz eines Öles als Trägerkomponente wurde durch die WO 97/03651 sogar ein Vorurteil geschaffen, sodaß deren erfolgreiche Herstellung äußerst überraschend und keinesfalls zu erwarten war.

Gegenstand der vorliegenden Erfindung ist demnach eine Emulsion vom Typ Öl in Wasser mit Schutzwirkung gegen Peroxidationsschäden an menschlichen Organen, insbesondere gegen Schäden durch Ischämie und/oder Reperfusion, zur i.v. Verabreichung, enthaltend, bezogen auf die Gesamtemulsion, α-Tocopherol in Form von all-rac-α-Tocopherol, dessen Stereoisomeren und/oder Gemischen einzelner Stereoisomerer in einer Menge entsprechend hinsichtlich Wirkung jener von 1,48 g/l bis 30 g/l RRR-α-Tocopherol, 0 bis 25 g/l eines i.v. verträglichen Öls, 0,25 g/l bis 3 g/l eines Phospholipids als Emulgator, sowie eine physiologische, nichtionische Substanz zur Einstellung der Osmolarität auf physiologische Werte und physiologische Mittel zur Einstellung des pH-Wertes auf 5 - 9.

Da die erfindungsgemäße Emulsion bevorzugt als Infusion an Patienten verabreicht wird, die durch eine vorhandene Mangeldurchblutung, gleichgültig ob diese durch ein Ereignis hervorgerufen ist, oder im Zuge eines operativen Eingriffes herbeigeführt werden mußte, belastet sind, und diese dann durch folgende chirurgische Eingriffe noch weiter belastet werden, ist es wünschenswert, die Belastung durch die Verabreichung der erfindungsgemäßen Emulsion so weit wie möglich einzuschränken. Bevorzugt ist bei den erfindungsgemäßen Emulsionen ein Gehalt an α-Tocopherol entsprechend 3,7 bis 22 g/l RRR-α-Tocopherol, wobei für jene Emulsionen, die neben α-Tocopherol ein i.v.verträgliches Öl enthalten, eine Menge desselben von 1 g/l bis 25 g/l bevorzugt ist wobei der Gehalt des als Emulgator verwendeten Phospholipids 1 g/l bis 3 g/l betragen soll. Wird auf den Zusatz von Öl verzichtet, ist bei gleichem α-Tocopherolgehalt ein Phospholipidgehalt von 0,25 g/l - 1,25 g/l zu bevorzugen.

Emulsionen ohne Verwendung eines i.v. verträglichen Öls sind als Infusion zur Verabreichung an Patienten in schlechtem oder gar kritischem Zustand besonders geeignet. Als α-Tocopherol wird vorzugsweise das all-rac-α-Tocopherol eingesetzt.

Als Ölkomponente können alle jene Öle eingesetzt werden, die bevorzugt in intravenös zu verabreichenden Fettemulsionen Anwendung finden. Als solche können insbesondere Sonnenblumenöl, Olivenöl, Maiskeimöl, Saffloröl, Baumwollsaatöl und Erdnußöl sowie Öle mit mittelkettigen Triglyceriden, aber auch diverse Fischöle genannt werden. Besonders bevorzugt ist Sojaöl. Als Phospholipide können Phosphatide pflanzlichen Ursprungs, insbesondere Sojaphosphatid eingesetzt werden, bevorzugt sind jedoch Eiphosphatide. Da eine optimale Verträglichkeit der erfindungsgemäßen Emulsionen angestrebt wird, ist es unerläßlich, deren Osmolarität auf physiologische Werte einzustellen. Als diesem Zweck dienende physiologische, nichtionische Substanzen dienen Zuckeralkohole, insbesondere Mannit und Glycerin, es können aber auch niedermolekulare Aminosäuren wie beispielweise Glycin verwendet werden. Die Verwendung von Glycerin ist hierbei bevorzugt. Es kann auch von Vorteil sein, die Stabilität der erfindungsgemäßen Emulsion durch Zusatz von langkettigen Fettsäuren zu erhöhen, deren Anteil bezogen auf die Gesamtemulsion maximal 0,6 % betragen soll. Ölsäure ist zu diesem Zweck besonders gut geeignet.

Sollte eine weitere Erhöhung der Stabilität der erfindungsgemäßen Emulsion gewünscht werden, kann dies durch weitere Zusätze, die stabilitätsfördernd wirken, erreicht werden. Als solche sind insbesondere zu nennen:
Dimyristoylphosphatidyl-glycerin, vorzugsweise in einer Menge von 0,025-0,24 g/l
Ubidecarenon, vorzugsweise 0,2 g/l - 0,6 g/l
Cholesterin, vorzugsweise 0,1 g/l - 0,5 g/l
In der oben zitierten Arbeit von Rabl et al., Kidney International 1993, (4), 912 - 917, wird ein wesentlicher Anteil der peroxidationshemmenden Wirkung dem relativ hohen Anteil an Ascorbat in der verabreichten wäßrig-organischen Lösung zugeschrieben. Vitamin C ist ein hydrophiles Antioxidans. Seine hemmende Wirkung auf die Lipidperoxidation beruht gemäß Rabl et al. auf einem Schutz von Vitamin E gegen Zerstörung durch freie Radikale, was einem Recycling von Vitamin E gleichkommt. Dies ist für die von Rabl et al. verwendete Lösung von besonderer Bedeutung, da in dieser der α-Tocopherolgehalt sehr gering ist und überdies dessen Hauptmenge als Acetat vorliegt. Gemäß vorliegender Erfindung können in der vorgesehenen Emulsionsform des Präparates wesentlich höhere Vitamin E Gehalte zur Anwendung kommen, sodaß einem Vitamin C Gehalt keine essentielle Wirkung zugeschrieben wird. Da Ascorbat für die Emulsion eine negative Auswirkung auf die Stabilität haben kann, wurde ein zwingender Gehalt an Ascorbat nicht vorgesehen. Wenn jedoch ein Mangel an biologisch reduzierenden Wasserstoffdonatoren (vor allem Vitamin C und Glutathion) vorliegt, kann ein Gehalt an Ascorbat günstige Auswirkungen haben. In solchen Fällen ist vorgesehen, Ascorbat der erfindungsgemäßen Emulsion als Ascorbylpalmitat zuzusetzen, da dies keinen wesentlichen Einfluß auf die Stabilität der Emulsion hat. Es wird bevorzugt in einer Menge von 12 - 25 g/l eingesetzt.

Zur Herstellung der erfindungsgemäßen Emulsion wird in der erprobten Weise vorgegangen, indem zunächst aus Wasser, dem Emulgator, der Substanz zur Einstellung der Osmolarität und, falls gewünscht, der langkettigen Fettsäure die wäßrige Phase in Form einer Suspension, mit einem schnellaufenden Rührer hergestellt wird, die durch Zugabe von NaOH auf den gewünschten pH-Wert von 7 - 9 eingestellt wird. Dieser Suspension wird dann die Ölphase zugesetzt, entweder in Form einer Mischung von α-Tocopherol in dem Öl oder das α-Tocopherol allein. Sollten zusätzliche Emulsionsstabilsatoren eingesetzt werden, werden diese der wäßrigen Suspension oder der Ölphase vor der Zumischung zur wäßrigen Phase zugesetzt. Diese Mischung wird dann mit dem gleichen schnellaufenden Rührer zu einer Voremulsion verarbeitet. Soll der erfindungsgemäßen Emulsion Ascorbylpalmitat zugesetzt werden, geschieht dies nach der Herstellung der Voremulsion, bevorzugt nach dem ersten Homogenisierungsschritt.

Als schnellaufende Rührer kommen Geräte wie Becomix oder der Ultraturrax in Frage. Beide Mischungsschritte erfolgen zweckmäßig bei erhöhter Temperatur, vorzugsweise bei 60 - 70 °C.

Die so erhaltene Voremulsion von wäßriger Phase und Ölphase wird dann einer Hochdruckemulgierung unterworfen, wobei ein Hochdruckhomogenisator oder ein Microfluidizer zum Einsatz kommen können. Die so erhaltene Emulsion wird in Flaschen abgefüllt und im Rotationsautoklaven hitzesterilisiert.

Die erfindungsgemäße Emulsion dient der Behandlung sämtlicher Formen von Ischämie mit darauffolgender Reperfusion, gleichgültig, ob sie durch ein Ereignis eingetreten ist oder bewußt herbeigeführt wurde. Grundlegendes Prinzip für die erfindungsgemäße Anwendung ist, daß sowohl die ganze Phase der Ischämie als auch der Reperfusion durch Erzeugung eines entsprechend hohen α-Tocopherolspiegels im Gewebe abgedeckt wird und die Verabreichung vor Beginn jedweden Eingriffes am Patienten erfolgt und zwar vorzugsweise so früh, daß genug Zeit fiir die Einstellung des optimalen Gewebsspiegels zur Verfügung steht. Das bringt neben der Errichtung der optimalen Voraussetzung für einen wirksamen Schutz vor Peroxidation auch den Vorteil mit sich, daß sich der Patient noch nicht im, durch die Operation verursachten Schockzustand befindet, was für die von Rabl et al. vorgesehene Verabreichung in der Ischämiephase, und zwar 30 Minuten vor Beginn der Reperfusion, nicht zutrifft.

Handelt es sich um einen operativen Eingriff an einem Patienten, bei dem eine Korrektur an einem der Organe auf chirurgischem Weg erfolgen soll, wie z.B. um eine Teilresektion an der Leber oder eine Operation am offenen Herzen, die eine Unterbindung der Durchblutung des entsprechenden Organs erforderlich machen, ist eine Verabreichung einer Einmaldosis 12 - 24 Stunden vor Operationsbeginn vorgesehen. Es ist aber auch möglich, mit der Applikation bereits 4 - 5 Tage vor der Operation zu beginnen und mehrere Dosen im Tagesabstand zu verabreichen, wobei die letzte Dosis vorzugsweise nicht später als 24 Stunden vor Operationsbeginn verabreicht werden soll. Dies empfiehlt sich, um in jedem Fall den optimalen Tocopherolspiegel im Gewebe des Patienten auch tatsächlich erzielt zu haben.

Handelt es sich bei dem operativen Eingriff um eine Organtransplantation, sollte die erfindungsgemäße Emulsion wenn möglich dem Spender und dem Empfänger verabreicht werden, wobei in beiden Fällen eine Applikation von zumindest 12 Stunden vor dem operativen Eingriff anzustreben ist. Sollte eine Verabreichung der erfindungsgemäßen Emulsion an den Spender nur ganz knapp vor der Organentnahme möglich sein, ist es dennoch angezeigt, die erfindungsgemäße Emulsion an Spender und Empfänger zu verabreichen und vorzugsweise auch der Organaufbewahrungslösung zuzusetzen. Dies empfiehlt sich auch, wenn ein langer Transportweg erforderlich ist, und zwar auch dann, wenn der Spender die erfindungsgemäße Emulsion rechtzeitig erhalten konnte. Besonders wichtig ist die Zugabe zur Organaufbewahrungslösung dann, wenn eine Verabreichung an den Spender nicht möglich war.

Die Dosen, die vorzugsweise verabreicht werden, betragen 2 - 50 mg/kg KG, insbesondere 10 - 40 mg/kg KG α-Tocopherol, die als Infusion, bevorzugt als einmalige Gabe, oder auch in bis zu 4 - 5 Tagesdosen geteilt gegeben werden. Diese Dosen gelten auch für Empfänger und Spender in der Organtransplantation, die beide die volle Dosis erhalten sollen.

Die protektive Wirkung der erfindungsgemäßen Emulsion wurde an verschiedenen Tiermodellen untersucht. In Modellversuchen an Ratten wurde die Überlebensrate nach partieller Leberischämie und -resektion geprüft. Mit dem Modell der Leberteilresektion kann die sogenannte 'warme' Ischämie untersucht werden. Dabei bleibt die physiologische Aktivität des Zielorgans trotz Ischämie (Unterversorgung mit Sauerstoff und Nährstoffen) aufrecht. Eine Schädigung, die durch die Ischämie hervorgerufen wird, tritt in diesem Modell somit sehr deutlich hervor. Im Gegensatz zur warmen Ischämie wird die physiologische Aktivität des Zielorgans bei der 'kalten' Ischämie reduziert und somit die Ischämietoleranz deutlich erhöht. Beispiele fiir eine kalte Ischämie sind u.a. Tiermodelle für Transplantationsversuche. Die Organschäden, die durch die Phase der Ischämie gesetzt wurden, sind bei dieser Form der Ischämie weniger deutlich und zeigen aufgrund der veränderten physiologischen Bedingungen auch einen anderen Zeitverlauf, da die Schädigungen oft erst im Verlauf der Reperfusion manifest werden.

Die Mortalität wurde am Modell der Leberteilresektion über 7 Tage nach dem Eingriff untersucht. Erfahrungsgemäß beträgt die Mortalität in diesem Modell nach einer partiellen Leberischämie von 60 Minuten 50%. Bei diesem Modell wird in einem operativen Eingriff durch temporäres Abklemmen der Pfortader und Arterienäste zu dem linken und mittleren Leberlappen (70% der Gesamtleber) in diesen Lappen eine partielle Leberischämie für die Dauer von 60 Minuten erzeugt. Nach dieser Zeit wird die Blutzufuhr durch Lösen des Verschlusses wieder freigegeben (Reperfusion). Anschließend wird der Rest der Leber, der in der Zwischenzeit normal durchblutet war, operativ (Resektion) entfernt. Die Tiere besitzen nur mehr die durch warme (Körpertemperatur) Ischämie geschädigten Leberlappen.

Die Applikation von 40 mg/kg α-Tocopherol mittels der erfindungsgemäßen Emulsion unmittelbar vor dem operativen Eingriff resultierte in einer verminderten Mortalität. Dieser Effekt war deutlich zeitabhängig. Der maximale Effekt wurde nach Applikation von 40 mg/kg α-Tocopherol mittels der erfindungsgemäßen Emulsion 24 Stunden vor dem operativen Eingriff erzielt. Bei diesem Versuch betrug die Überlebensrate 100%. In einem weiteren operativ gleichartigen Versuch wurden die Versuchstiere 3 Stunden nach Beginn der Reperfusionsphase getötet und die Leberschädigung in Lebergewebebiopsien und an Hand von Leberenzymen untersucht.

Als Marker fiir die Organschäden wurden in diesen Versuchen die Serumspiegel der Transaminasen (GOT, GPT, GIDH und LDH) gemessen. Analog zu den Überlebensversuchen zeigte sich, daß der protektive Effekt deutlich zeitabhängig war. Bei der Applikation der erfindungsgemäßen Emulsion 24 Stunden vor dem operativen Eingriff war der Effekt maximal. Analoge Ergebnisse wurden in diesem Modell in dem Gewebe der Leberbiopsien für die Lipidperoxidation (TBARS) gefunden.

Im Modell der orthotopen Lebertransplantation wird das transplantierte Organ in räumlicher Übereinstimmung zu dem ersetzten Organ eingesetzt. An diesem Modell fiir die 'kalte' Ischämie (das Spenderorgan wird nach Entnahme in einer gekühlten Lösung zur Organkonservierung bis zur Transplantation aufbewahrt) konnte in Versuchen an Ratten der Anstieg der Transaminasen sowie das Ausmaß der Lipidperoxidation nach Applikation der erfindungsgemäßen Emulsion noch deutlicher reduziert werden. Die Reduktion der Transaminasen betrug im Mittel etwa 75%, das Ausmaß der Lipidperoxidation, gemessen am MDA-Wert, wurde um mehr als 50% reduziert.

Die Dosisabhängigkeit wurde am Modell der partiellen Leberischämie untersucht. Die geschilderten Effekte waren direkt dosisabhängig, wobei besonders im Bereich zwischen 10 und 40 mg/kg α-Tocopherol relevante Reduktionen der Transaminasen beobachtet wurden.

Aus diesen Versuchen lassen sich die folgenden Schlußfolgerungen ziehen: durch die Verabreichung der erfindurigsgemäßen Emulsion läßt sich eine Eingrenzung des Ischämie/Reperfusionsschadens der Leber nach kalter und nach warmer Ischämie erzielen. Bei kalter Ischämie (Transplantation) werden die Endothelzellen geschützt, während bei warmer Ischämie die Leberzellen direkt geschützt werden. Eine ausreichende Auffsättigung der α-Tocopherol Gewebespiegel durch präoperative Supplementation ist durch die Applikation der erfindungsgemäßen Emulsion gewährleistet. Die Organfunktion wird einerseits durch den direkten protektiven Effekt auf die Leberzellen postoperativ und andererseits durch eine verbesserte Mikrozirkulation durch die Protektion des Gefäßendothels verbessert. Die Gabe der erfindungsgemäßen Emulsion hat weiters einen positiven Einfluß auf die Zellschädigung nach Ischämie/Reperfusion durch einen direkten antioxidativen Effekt gemessen an der Reduktion der Lipidperoxidation und durch einen anti-apoptotischen Effekt im Modell der orthotopen Lebertransplantation.

### Beispiele:

**Beispiel 1:** 25 g wasserfreies Glycerin werden in 220 ml Wasser ad injectionem bei einer Temperatur von 60 bis 70 °C in einem Becomix gemischt und mit einer Suspension von 3 g Eilezithin (E-80, Firma: Lipoid), 0,2 g Ölsäure, etwa 0,7 ml IN Natronlauge zur Einstellung eines pH-Wertes von 8 bis 9 und 80 ml Wasser versetzt. 5,4 g all-rac-α-Tocopherolentsprechend 4 g RRR-a-Tocopherol werden mit 25 g Sojabohnenöl gemischt, die Mischung wird auf 60 - 70 °C vorgewärmt, in die wäßrige Suspension eingetragen und bei einem Druck von 350 bar in einem Hochdruckhomogenisator in 4 Stufen homogenisiert. Die entstandene Emulsion wird mit Wasser ad injectionem auf 1000 ml aufgefüllt, in 100 ml Glasflaschen abgefüllt und bei 121 °C im Rotationsautoklaven hitzesterilisiert. Diese 100 ml Flaschen stellen beispielsweise eine Tagesdosis für die Anwendung bei Transplantationen, z.B. von Leber oder Nieren, am Menschen dar.

**Beispiel 2:** 25 g wasserfreies Glycerin, 220 ml Wasser ad injectionem, 3 g Eilezithin, 0,2 g Ölsäure und etwa 0,7 ml IN Natronlauge, die auf 60 - 70 °C angewärmt sind, werden nach und nach in einen schnellaufenden Rührer (z.B. Ultra Turrax) eingetragen und zu einer gleichmäßigen Suspension verarbeitet. 27 g all-rac-α-Tocopherol entsprechend 20 g RRR-a-Tocopherol werden mit 25 g Sojabohnenöl vorgemischt und in die im Rührgefäß vorgelegte wäßrige Suspension eingetragen. Mittels des schnellaufenden Rührers wird daraus eine Voremulsion bereitet. Diese wird dann in einem Microfluidizer bei 600 bar in 5 Stufen homogenisiert. Nach Auffüllen auf 1000 ml mit Wasser ad injectionem wird in 50 ml Flaschen abgefüllt und wie in Beispiel 1 beschrieben sterilisiert.

**Beispiel 3:** 25 g wasserfreies Glycerin, 0,1 g Ölsäure und 1,0 g Eilezithin werden mit 300 ml Wasser, das auf einen pH -Wert von 8 - 9 eingestellt ist, in einem schnell laufenden Rührer bei 60 - 70 °C zu einer Suspension verarbeitet. In diese Mischung werden direkt 5,4 g all-rac-α-Tocopherol entsprechend 4 g RRR-α-Tocopherol eingetragen und mit dem schnell laufenden Rührer zu einer Voremulsion verarbeitet. Die Homogenisierung wird, wie in Beispiel 2 beschrieben, vorgenommen. Nach Auffüllen auf 1 1 mit Wasser ad injectionem, Abfüllen in 100 ml Flaschen und Hitzesterilisation wie in Beispiel 1 beschrieben, ist die Emulsion für die Verminderung von Oxidationsschäden, besonders bei empfindlichen Patienten geeignet.

**Beispiel 4:** 22,5 g wasserfreies Glycerin, 220 ml Wasser ad injectionem, 2 g Eilezithin, 0,2 g Ölsäure, 0,4 g Ubidecarenon und etwa 0,7 ml IN Natronlauge, die auf 60- 70 °C angewärmt sind, werden nach und nach in einen schnellaufenden Rührer (z.B. Ultra Turrax) eingetragen und zu einer gleichmäßigen Suspension verarbeitet. 3 g all-rac-α-Tocopherol entsprechend 2,2 g RRR-α-Tocopherol werden mit 12 g Sojabohnenöl vorgemischt und in die im Rührgefäß vorgelegte wäßrige Suspension eingetragen. Mittels des schnellaufenden Rührers wird daraus eine Voremulsion bereitet. Diese wird dann in einem Microfluidizer bei 600 bar in 5 Stufen homogenisiert. Nach Auffüllen auf 1000 ml mit Wasser ad injectionem wird abgefüllt und wie in Beispiel 1 beschrieben sterilisiert.

**Beispiel 5:** 22,5 g wasserfreies Glycerin, 220 ml Wasser ad injectionem, 2,5 g Eilezithin, 0,6 g Ölsäure und etwa 0,7 ml IN Natronlauge, die auf 60 - 70 °C angewärmt sind, werden nach und nach in einen schnellaufenden Rührer (z.B. Ultra Turrax) eingetragen und zu einer gleichmäßigen Suspension verarbeitet. 10 g all-rac-α-Tocopherol entsprechend 7,4 g RRR-α-Tocopherol werden mit 20 g Sojabohnenöl vorgemischt und in die im Rührgefäß vorgelegte wäßrige Suspension eingetragen. Mittels des schnellaufenden Rührers wird daraus eine Voremulsion bereitet. Diese wird dann in einem Microfluidizer bei 600 bar einmal homogenisiert. In diese Grobemulsion werden 23,4 g Ascorbylpalmitat gelöst und danach noch 4 mal bei 600 bar homogenisiert. Die so erhaltene Emulsion wird wie in Beispiel 4 beschrieben weiterverarbeitet.

## Patentansprüche

1. Emulsion vom Typ Öl in Wasser mit Schutzwirkung gegen Peroxidationsschäden an menschlichen Organen, insbesondere gegen Schäden durch Ischämie und/oder Reperfusion, zur i.v.Verabreichung, enthaltend, bezogen auf die Gesamtemulsion, α-Tocopherole in Form von all-rac-α-Tocopherol, dessen Stereoisomeren und/oder Gemische einzelner Stereoisomerer in einer Menge, entsprechend hinsichtlich Wirkung jener von 1,48 g/L bis 30 g/L RRR-α-Tocopherol, 0 bis 25 g/L eines i.v. verträglichen Öls, 0,25 - 3 g/L eines Phospholipids als Emulgator, sowie eine physiologische, nichtionische Substanz zur Einstellung der Osmolarität auf physiologische Werte und physiologische Mittel zur Einstellung des pH-Wertes auf 7 - 9.

2. Emulsion gemäß Anspruch 1, enthaltend das α-Tocopherol in einer Menge, entsprechend hinsichtlich Wirkung jener von 3.7 g/L - 22 g/L RRR-α-Tocopherol, das Öl in einer Menge von 1 g/L bis 25 g/L und das Phospholipid in einer Menge von 1-3 g/L.

3. Emulsion gemäß Anspruch 1 mit α-Tocopherol als einziger Lipidkomponente, enthaltend das α-Tocopherol in einer Menge, entsprechend hinsichtlich Wirkung jener von 3,7 g/L - 22 g/L RRR-α-Tocopherol und das Phospholipid in einer Menge von 0,25 - 1,25 g/L.

4. Emulsion gemäß den Ansprüchen 1, 2 oder 3, **dadurch gekennzeichnet, dass** das α-Tocopherol all-rac-α-Tocopherol, das Öl Sojaöl, das Phospholipid Eilezithin und das Mittel zur Einstellung der Osmolarität Glycerin ist.

5. Emulsion gemäß den Ansprüchen 1 bis 4, **dadurch gekennzeichnet, dass** sie bis 0,6 % Gewicht/Volumen, bezogen auf die Gesamtemulsion, einer langkettigen Fettsäure enthält.

6. Emulsion gemäß den Ansprüchen 1 - 5, **dadurch gekennzeichnet, dass** sie zusätzlich Mittel zur Erhöhung der Emulsionsstabilität aus der Gruppe
0,025 g/L - 0,24 g/L Dimyristoylphosphatidylglycerin,
0,2 g/L - 0,6 g/L Ubidecarenon und
0,1 g/L - 0,5 g/L Cholesterin enthält.

7. Emulsion gemäß den Ansprüchen 1 bis 6, **dadurch gekennzeichnet, dass** sie zusätzlich 12 - 25 g/L Ascorbyl-Palmitat enthält.

8. Verfahren zur Herstellung der Emulsion gemäß den Ansprüchen 1 bis 7, **dadurch gekennzeichnet, dass** das Phospholipid mit Wasser, das auf einen pH-Wert von 7 - 9 eingestellt ist, mit der physiologischen, nichtionischen Substanz zur Einstellung der Osmolarität und gewünschtenfalls mit der langkettigen Fettsäure und/oder dem Ascorbyl-Palmitat zu einer wäßrigen Suspension verarbeitet wird, der das α-Tocopherol als solches oder in Form einer Vormischung mit dem i.v.verträglichen Öl sowie mit gegebenenfalls vorgesehenen Mitteln zur Erhöhung der Emulsionsstabilität zugemischt wird, worauf die resultierende Mischung einer Hochdruckhomogenisierung unterworfen wird.

9. Verwendung der Emulsion gemäß mindestens einem der Ansprüche 1-7 zur Herstellung einer Infusion zur Verminderung von Peroxidationsschäden durch Ischämie und Reperfusion während einer Organtransplantation, die für die Verabreichung an den Spender und an den Empfänger vorgesehen ist.

10. Verwendung der Emulsion gemäß mindestens einem der Ansprüche 1-7 zur Herstellung eines Zusatzes zur Aufbewahrungslösung für das zur Transplantation vorgesehene Organ.

## Claims

1. Oil-in-water emulsion exhibiting a protective action against damage to human organs resulting from peroxidation, especially damages caused by ischaemia and/or reperfusion, for i.v. administration and containing, relative to the total emulsion, α-tocopherols in the form of all-rac-α-tocopherol, its stereoisomers and/or mixtures of individual stereoisomers in a quantity corresponding, with regard to effect, to that of 1.48 g/L to 30 g/L RRR- α-tocopherol, 0 to 25 g/L of an i.v. compatible oil, 0.25 - 3 g/L of a phospholipid as emulsifier and a physiological, non-ionic substance for adjustment of the osmolarity to physiological values and physiological means for adjustment of the pH value to 7 - 9.

2. Emulsion according to claim 1 containing the α-tocopherol in a quantity corresponding, with regard to effect, to that of 3.7 g/L - 22 g/L RRR α-tocopherol, the oil in a quantity of 1 g/L to 25 g/L and the phospholipid in a quantity of 1 - 3 g/L.

3. Emulsion according to claim 1, with α-tocopherol as sole lipid component, containing the α-tocopherol in a quantity corresponding, with regard to effect, to that of 3.7 g/L -22 g/L RRR-α tocopherol and the phospholipid in a quantity of 0.25 - 1.25 g/L.

4. Emulsion according to claims 1, 2 or 3, **characterised in that** the α-tocopherol is all-rac-α-tocopherol, the oil is soya oil, the phospholipid is egg lecithin and the material for adjustment of the osmolarity is glycerine.

5. Emulsion according to claims 1 to **4, characterised in that** it contains up to 0.6% weight/volume, relative to the total emulsion, of a long-chained fatty acid.

6. Emulsion according to claims 1 - 5, **characterised in that** it additionally contains means to increase the emulsion stability, selected from the group
0.025 g/L - 0.24 g/L dimyristoylphosphatidyl glycerine,
0.2 g/L - 0.6 g/L Ubidecarenon and
0.1 g/L - 0.5 g/L cholesterol.

7. Emulsion according to claims 1 to 6, **characterised in that** it additionally contains 12 - 25 g/L ascorbyl-palmitate.

8. Method for the production of the emulsion according to claims 1 to 7, **characterised in that** the phospholipid is processed with water which is adjusted to a pH value of 7 - 9, with the physiological, nonionic substance for adjustment of the osmolarity and, if desired, with the long-chained fatty acid and/or the ascorbic palmitate to an aqueous suspension to which is admixed the α-tocopherol as such or in the form of a premixture with the i.v. compatible oil and with, possibly, means to increase the stability of the emulsion, whereupon the resultant mixture is subjected to high-pressure homogenisation.

9. Use of the emulsion in accordance with at least one of the claims 1 - 7 for the preparation of an infusion to lessen the damage resulting from peroxidation caused by ischaemia and reperfusion and which is provided for administration to the donor and to the recipient.

10. Use of the emulsion in accordance with at least one of claims 1 - 7 for the preparation of an additive to the preservation solution for the organ provided for transplantation.

## Revendications

1. Emulsion du type huile dans l'eau possédant un effet de protection contre des dégâts causés à des organes humains par la peroxydation, en particulier contre des dégradations provoquées par une ischémie et/ou par une reperfusion, destinée à une administration par voie intraveineuse, contenant, rapportés à l'émulsion totale, des α-tocophérols sous la forme du all-rac-α-tocophérol, de ses stéréo-isomères et/ou de mélanges de stéréo-isomères individuels en une quantité qui correspond, en ce qui concerne l'activité, à celle de 1,48 g/litre à 30 g/litre du RRR-α-tocophérol, de 0 à 25 g/litre d'une huile compatible par voie intraveineuse, de 0,25 à 3 g/litre d'un phospholipide à titre d'émulsifiant, ainsi qu'une substance physiologique non ionique pour le réglage de l'osmolarité à des valeurs physiologiques, et les agents physiologiques pour le réglage de la valeur du pH à 7 - 9.

2. Emulsion selon la revendication 1, contenant le tocophérol en une quantité qui correspond, en ce qui concerne l'activité, à celle de 3,7 g/litre à 22 g/litre du RRR-α-tocophérol, l'huile en une quantité de 1 g/litre à 25 g/litre et le phospholipide en une quantité de 1 à 3 g/litre.

3. Emulsion selon la revendication 1, comprenant de l'α-tocophérol à titre de composant lipidique unique, qui contient l'α-tocophérol en une quantité qui correspond, en ce qui concerne l'activité, à celle de 3,7 g/litre à 22 g/litre du RRR-α-tocophérol, et le phospholipide en une quantité de 0,25 à 1,25 g/litre.

4. Emulsion selon les revendications 1, 2 ou 3, **caractérisée en ce que** l'α-tocophérol est le all-rac-a-tocophérol, l'huile est de l'huile de soja, le phospholipide est de la lécithine d'oeuf et l'agent pour le réglage de l'osmolarité est du glycérol.

5. Emulsion selon la revendication 1 à 4, **caractérisé en ce qu'**elle contient, jusqu'à concurrence de 0,6 % en poids/volume, rapporté à l'émulsion totale, un acide gras à longue chaîne.

6. Emulsion selon les revendications 1 à 5, **caractérisée en ce qu'**elle contient, des agents supplémentaires pour augmenter la stabilité de l'émulsion, choisis parmi le groupe comprenant
du dimyristoylphosphatidylglycérol à raison de 0,025 g/litre - 0,24 g/litre ;
de l'ubidécarénone à raison de 0,2 g/litre - 0,6 g/litre ; et
du cholestérol à raison de 0,1 g/litre -- 0,5 g/litre.

7. Emulsion selon les revendications 1 à 6, **caractérisée en ce qu'**elle contient en outre du palmitate d'ascorbyle à raison de 12 à 25 g/litre.

8. Procédé pour la préparation de l'émulsion selon les revendications 1 à 7, **caractérisé en ce qu'**on traite le phospholipide avec de l'eau, qui a été réglée à une valeur de pH de 7 à 9, avec la substance physiologique non ionique pour régler l'osmolarité et le cas échéant avec l'acide gras à longue chaîne et/ou avec le palmitate d'ascorbyle pour obtenir une suspension aqueuse à laquelle on ajoute par mélange l'α-tocophérol comme tel ou sous la forme d'un prémélange avec l'huile compatible par voie intraveineuse, ainsi qu'avec des agents le cas échéant prévu pour augmenter la stabilité de l'émulsion, avant de soumettre le mélange résultant à une homogénéisation sous haute pression.

9. Utilisation de l'émulsion selon au moins une des revendications 1 à 7 pour préparer une perfusion destinée à diminuer les dégâts de peroxydation causés par une ischémie et par une reperfusion au cours d'une transplantation d'organe, ladite perfusion étant prévue pour être administrée au donneur et au receveur.

10. Utilisation de l'émulsion selon au moins une des revendications 1 à 7, pour la préparation d'un additif pour la solution de conservation destinée à l'organe prévu pour la transplantation.
